# EUROPEAN PATENT APPLICATION

(11) **EP 1 477 555 A2**
(43) Date of publication of application: **17.11.2004**
(21) Application number: 04252818.2
(22) Date of filing: 14.05.2004
(51) Int. Cl.: C12N 1/20, A61K 35/74

(54) **Method for culturing bacterial cells belonging to the enterococcus genus and method of producing killed bacterial cells belonging to the enterococcus genus**

(30) Priority: 15.05.2003 JP 2003136673
(71) Applicant: Kawai, Yasuo, Ito-shi, Shizuoka 413-0233 (JP)
(72) Inventor: Kawai, Yasuo, Ito-shi, Shizuoka 413-0233 (JP)
(74) Representative: Smaggasgale, Gillian Helen

(57) **Abstract**

A method of culturing is provided which keeps physiology activity of bacterial cell belonging to the *Enterococcus* genus and also a method of producing killed bacterial cells belonging to the *Enterococcus* genus.

In the method of culturing bacterial cells belonging to the *Enterococcus* genus, the method cultures bacterial cells belonging to the *Enterococcus* genus in an aerobic manner under the culturing conditions of (i) pH: 5.5 to 7.5, (ii) stirring method: 5 to 40 times per minute, (iii) temperature conditions: 30 to 40°C, and (iv) culturing time: 6 to 24 hours, and extracts viable bacteria from culture solution.

## Description

### BACKGROUND OF THE INVENTION

### Field of the Invention

The present invention relates to a method of culturing bacterial cells of the *Enterococcus* genus, a method of producing killed bacterial cells of the *Enterococcus* genus, and to their use.

### Description of Prior Art

With the recent increase of consumption of animal fat per Japanese, and the progress towards Europe and America of the meal life, the number of lifestyle diseases, such as hyperlipidemia which is an adult ailment, and the number of patients with atherosclerosis are increasing.

Therefore, a variety of medical drugs for treatment of hyperlipidemia have been put to practical use. However, it has been reported that all of the drugs which are conventionally used for suppressing the disease have side effects such as stomach discomfort, vomiting, abnormal liver function, acute kidney insufficiency, a feeling of lassitude, or the like after being taken, and further, sometimes, persons who are taking other drugs may have to stop taking them.

The inventor of the present application discovered, as a result of study of a new anti-atherosclerosis agent, that bacterial cells, particularly viable bacteria and killed bacterial cells of a variety of microorganisms belonging to the *Enterococcus* genus cause the blood cholesterol value and the triglyceride value to reduce remarkably, and obtained knowledge that since the origin of the microorganisms is intestinal bacteria which are originally present internally of the human body, even if ingested, they are nonpoisonous, and there is no side effects like the conventional anti-atherosclerosis agents or the medical drugs for treatment of hyperlipidemia (as described, for example, in Japanese Patent No. 2756778).

Further, the inventor showed, as one of knowledge, that these microorganisms have a variety of physiological activities; however, these have the activity for causing the cholesterol value and the triglyceride value to reduce remarkably as one example of physiological activity thereof, not only in the form of viable bacteria but also in the form of killed bacterial cells processed under the fixed conditions.

### SUMMARY OF THE INVENTION

However, thus, the inventor showed that even if it is the bacterial cells belonging to the *Enterococcus* genus having the activity reducing the cholesterol value and the triglyceride value remarkably, the activity of the bacterial cells reduced and the effect expected is not obtained depending on the method of culturing. Further, killed bacterial cells belonging to the *Enterococcus* genus are also known to reduce the cholesterol value and the triglyceride value remarkably; however, it has been shown that the effect expected is lost depending on the method of getting the killed bacterial cells from the viable bacteria.

The present invention relates to a method of culturing which results in no loss of the activity of bacterial cells belonging to *the Enterococcus* genus having the effect to reduce blood cholesterol and triglyceride, and a method of producing killed bacterial cells belonging to the *Enterococcus* genus.

Therefore, the present application provides a method of culturing bacterial cells belonging to the *Enterococcus* genus, having the activity of reducing cholesterol and triglyceride, the method comprising: culturing the *Enterococcus* genus cells in an aerobic manner under the culturing conditions of (i) pH : 5.5 to 7.5, (ii) stirring method: 5 to 40 times per minute, (iii) temperature conditions: 30 to 40°C, and (iv) culturing time: 6 to 24 hours, and extracting viable bacteria from the culture solution.

Preferably, the culture medium composition in the culturing step contains (i) glucose: 1 to 4%, (ii) yeast extract: 3 to 6%, (iii) peptone: 0.1 to 5%, and (iv) dibasic potassium phosphate (K₂HPO₄): 1 to 5%.

Preferably also, the *Enterococcus* genus bacterial cells are viable bacteria selected from *Enterococcus faecalis, Enterococcus faecium, Enterococcus bovis, Enterococcus avium, Enterococcus durans, Enterococcus salivarius, Enterococcus mitis,* and *Enterococcus equines,* and mixtures of two or more thereof.

The present invention further provides a method of producing killed bacterial cells belonging to the *Enterococcus* genus comprising:
(1) culturing bacterial cells of the *Enterococcus* genus in an aerobic manner under the following culturing conditions:
   (i) a pH of 5.5 to 7.5;
   (ii) a stirring rate of 5 to 40 revolutions per minute;
   (iii) a temperature of 30 to 40°C; and
   (iv) a culturing period of from 6 to 24 hours;
(2) washing resulting viable bacterial cells with physiological aqueous saline solution; and
(3) treating a bacterial liquid containing said viable bacterial cells suspended in a physiological aqueous saline solution under the following conditions:
   (a) a temperature of from 80 to 125°C;
   (b) a pressure of from 5 to 25 pounds per square inch [34.73 to 172.37 kPa]; and
   (c) a period of from 5 to 25 minutes; thereby to kill said viable bacterial cells.

In this case, the culture medium composition in said culturing step (1) may contain (i) glucose : 1 to 4%, (ii) yeast extract: 3 to 6%, (iii) peptone : 0.1 to 5%, and (iv) dibasic potassium phosphate (K₂HPO₄) : 1 to 5%.

### BEST MODE FOR CARRYING OUT OF THE INVENTION

In the following description, the kind of microorganisms that can be used in the method of culturing bacterium cells belonging to the *Enterococcus* genus and the method of producing killed bacterial cells belonging to the *Enterococcus* genus according to the present invention, the culturing method for the microorganisms, the method of producing killed bacterial cells of the cultured microorganisms and the medical action of bacterial cells and killed bacterial cells obtained by the methods of the invention will be described in detail.

### 1. Microorganisms

Bacterial cells of the *Enterococcus* genus are used, and particularly, there are illustrated, as suitable material, viable bacteria selected from a group comprising *Enterococcus faecalis, Enterococcus faecium, Enterococcus bovis, Enterococcus avium, Enterococcus durans, Enterococcus salivarius, Enterococcus mitis,* and *Enterococcus equines,* and mixtures of two or more thereof. Further, bacterial cells belonging to the above-described *Enterococcus* genus can be obtained, for example, under Accession Number ATCC700802 from ATCC (American Type Culture Collection).

### 2. Conditions for producing the viable bacteria

The preferred conditions for culturing the viable bacteria are as follows:
(i) Hydrogen ion exponent (pH): the range from 5.5 to 7.5.
(ii) Stirring method: gently stir 5 to 40 times per minute.
(iii) Culturing time: the range from 6 to 24 hours.
(iv) Culture medium compositions: glucose; 1 to 4%, yeast extract; 0.3 to 6%, peptone; 0.1 to 5%, and potassium phosphate (K₂HPO₄) ; 1 to 5%.
(v) Temperature: under the temperature conditions of 30 to 45°C.
(vi) It is considered that aerobic culturing is carried out, but addition of enzyme is not particularly necessary.

### 3. Conditions for producing the killed bacterial cells

The viable bacterial culture obtained on the basis of the vital bacteria producing conditions described in section 2 above is further washed about one to two times with physiological brine, and then suspended in an aqueous physiological salt solution to obtain a bacterial liquid, which liquid is subjected to heating and pressure processing under the conditions mentioned below:
(i) Temperature: 80 to 125°C
(ii) Pressure: 5 to 25 pounds per square inch [34.73 to 172.37 kPa]
(iii) Time: 5 to 25 minutes

The actively killed bacterial cells of said microorganisms can be obtained by the heating and high pressure processing in accordance with the aforementioned conditions.

### 4. Medical action

As shown in Experimental Examples described below, it is shown that, by the method of producing killed bacterial cells according to the present invention, that the *Enterococcus* genus causes the blood cholesterol and triglyceride values to reduce extremely effectively.

### (1) Experimental Example 1

The viable bacteria of various microorganisms belonging to the *Enterococcus* genus shown in Section 1 above and the heating and pressure processed bacteria were orally administered, in an amount corresponding to 10¹⁰/day of viable bacteria and 10¹⁰/day of killed bacterial cells, every day for four weeks to mice (male, 18 weeks old, average weight 20g: 10 mice in each group) to measure the reducing rate of cholesterol in a serum. The results of this experiment are shown in Table 1.

**TABLE 1**

| Comparison of the cholesterol reducing rate between viable cells and killed cells (mouse) | | |
|---|---|---|
| Kind of microorganisms | Viable cells (%) | Killed cells (%) |
| *E. faecalis* K-23 | 19.5 | 44.0 |
| *E. faecalis* K-10 | 17.5 | 32.2 |
| *E. faecalis* K-57 | 18.3 | 30.4 |
| *E. faecium* K-58 | 19.0 | 32.5 |
| *E. avium* K-56 | 16.4 | 28.7 |
| *E. avium* K-57 | 15.8 | 23.6 |
| *E. avium* K-59 | 16.5 | 27.3 |
| *E. durans* K-66 | 12.7 | 22.8 |
| *E. durans* K-67 | 13.2 | 21.0 |
| *E. bovis* K-76 | 14.6 | 22.5 |

### (2) Experimental Example 2

Similarly to the above-described Experimental Example 1, viable bacteria of various microorganisms belonging to the *Enterococcus* genus and the heating and pressure processed killed bacterial cells were orally administered, in the amount corresponding to 10¹⁰/day of viable bacteria and 10¹⁰/day of killed bacterial cells, every day for four weeks to normal rats (male, 18 weeks old, average weight 250g: 7 rats in each group) to measure the reducing rate of cholesterol in a serum. The results of this experiment are shown in Table 2.

**TABLE 2**

| Kind of microorganisms | Viable cells (%) | Killed cells (%) | | |
|---|---|---|---|---|
| | Normal rat | Normal rat | Normal rat (*1) | Normal rat (*2) |
| *E. faecalis* K-23 | 13.0 | 28.1 | 38.1 | 29.3 |
| *E. faecalis* K-10 | 11.0 | 20.0 | 22.8 | 18.2 |
| *E. faecalis* K-57 | 11.4 | 19.6 | 21.4 | 19.0 |
| *E. faecium* K-58 | 9.6 | 17.5 | 17.6 | 15.3 |
| *E. avium* K-56 | 8.4 | 16.7 | 17.2 | 14.8 |
| *E. avium* K-57 | 8.2 | 16.5 | 16.8 | 14.5 |
| *E. avium* K-59 | 8.2 | 17.3 | 17.5 | 13.3 |
| *E. durans* K-66 | 9.5 | 17.7 | 18.0 | 15.1 |
| *E. durans* K-67 | 9.4 | 17.0 | 17.8 | 14.7 |
| *E. bovis* K-76 | 9.8 | 17.5 | 18.2 | 15.2 |

| | | | | |
|---|---|---|---|---|
| (*1) Cholesterol load diet rat | | | | |
| (*2) Fructose load diet rat | | | | |

### (3) Experimental Example 3

The viable bacteria of various microorganisms belonging to the *Enterococcus* genus and the heating and pressure processed killed bacterial cells, regulated in accordance with the above-described viable bacteria regulating Example and killed bacterial cell regulating Example, were orally administered, in an amount corresponding to 10¹⁰/day of viable bacteria and 10¹⁰/day of killed bacterium cells, every day for four weeks to normal mice (18 weeks old, average weight 20g: 10 mice in each group) to measure the reducing rate of triglyceride in a serum. The results of this experiment are shown in Table 3.

**TABLE 3**

| Kind of microorganisms | Viable cells (%) | Killed cells (%) |
|---|---|---|
| *E. faecalis* K-23 | 17.1 | 76.1 |
| *E. faecalis* K-10 | 19.0 | 43.2 |
| *E. faecalis* K-57 | 17.3 | 37.0 |
| *E. faecium* K-58 | 17.3 | 66.4 |
| *E. avium* K-56 | 17.9 | 40.1 |
| *E. avium* K-57 | 15.1 | 33.2 |
| *E. avium* K-59 | 9.7 | 30.4 |
| *E. durans* K-66 | 9.8 | 55.1 |
| *E. durans* K-67 | 9.9 | 74.2 |
| *E. bovis* K-76 | 9.5 | 61.6 |

### (4) Experimental Example 4

The viable bacteria of various microorganisms belonging to the *Enterococcus* genus and the heating and pressure processed killed bacterial cells, regulated similarly to the above-described Experimental Example 3, were orally administered, in an amount corresponding to 10¹⁰/day of viable bacteria and 10¹⁰/day of killed bacterial cells, every day for four weeks to normal rats (male, 18 weeks old, average weight 250g: 7 rats in each group) to measure the reducing rate of triglyceride in a serum. The results of this experiment are shown in Table 4.

**TABLE 4**

| Kind of microorganisms | Viable cells (%) | Killed cells (%) | | |
|---|---|---|---|---|
| | Normal rat | Normal rat | Normal rat (*1) | Normal rat (*2) |
| *E. faecalis* K-23 | 34.9 | 61.7 | 54.8 | 69.1 |
| *E. faecalis* K-10 | 25.4 | 36.5 | 49.3 | 62.7 |
| *E. faecalis* K-57 | 34.5 | 44.3 | 41.6 | 47.8 |
| *E. faecium* K-58 | 18.5 | 43.5 | 39.6 | 46.5 |
| *E. avium* K-56 | 19.0 | 33.4 | 34.8 | 43.5 |
| *E. avium* K-57 | 21.4 | 32.6 | 36.5 | 36.4 |
| *E. avium* K-59 | 13.6 | 39.7 | 38.7 | 31.0 |
| *E. durans* K-66 | 13.3 | 37.4 | 43.9 | 48.7 |
| *E. durans* K-67 | 12.2 | 22.8 | 28.6 | 33.6 |
| *E. bovis* K-76 | 18.7 | 37.8 | 36.3 | 40.4 |

| | | | | |
|---|---|---|---|---|
| (*1) Cholesterol load diet rat | | | | |
| (*2) Fructose load diet rat | | | | |

### (5) Safety of microorganisms belonging to the Enterococcusgenus

In the acute toxicity test, *Enterococcus faecalis* K-23 heating and pressure processed bacteria in as large an amount as possible were orally administered to rats (male, 18 weeks old, average weight 250 g: 10 rats in a group) (5600 mg/kg body weight), but no abnormality was recognized, and in the oral administration, in fact, there was no poisonous effect. Further, also in the intraperitoneal administration (male mice, 18 weeks old, weight 20g: 10 mice in each group), there was high in value, e.g. 802 mg/kg body weight. The experimental result on the safety is shown in Table 5.

**TABLE 5**

| Safety of *Enterococcus faecalis* KAWAI | | |
|---|---|---|
| *E. faecalis* | Killed cells Oral administration | >5600mg/kg body weight |
| | Killed cells Intraperitoneral administration | >802mg/kg body weight |

As described above, in the method of culturing bacterial cells belonging to the *Enterococcus* genus of the present invention, the method cultures the bacterium cells belonging to the *Enterococcus* genus in an aerobic manner under the culturing conditions of (i) pH : 5.5 to 7.5, (ii) stirring method : 5 to 40 times per minute, (iii) . temperature conditions : 30 to 40, and (iv) culturing time : 6 to 24 hours, and extracts viable bacteria from the culture solution.

Further, the killed bacterial cells of the bacterium cells are obtained by
(1) culturing bacterial cells of the Enterococcus genus in an aerobic manner under the following culturing conditions:
   (i) a pH of 5.5 to 7.5;
   (ii) a stirring rate of 5 to 40 revolutions per minute;
   (iii) a temperature of 30 to 40°C; and
   (iv) a culturing period of from 6 to 24 hours;
(2) washing resulting viable bacterial cells with physiological aqueous saline solution; and
(3) treating a bacterial liquid containing said viable bacterial cells suspended in a physiological aqueous saline solution under the following conditions:
   (a) a temperature of from 80 to 125°C;
   (b) a pressure of from 5 to 25 pounds per square inch [34.73 to 172.37 kPa]; and
   (c) a period of from 5 to 25 minutes; thereby to kill said viable bacterial cells.

Therefore, the present invention established the method of culturing bacterial cells belonging to the *Enterococcus* genus having effect to reduce the blood cholesterol and the triglyceride, and the method of producing the killed bacterial cells thereof.

The present invention provides the method of culturing keeping the physiology activity of the bacterial cells belonging to the *Enterococcus* genus and the method of producing the killed bacterial cells belonging to the *Enterococcus* genus, and has applicability in the industrial field.

## Claims

1. A method of culturing bacterial cells belonging to the *Enterococcus* genus, the method comprising: culturing bacterial cells belonging to the *Enterococcus* genus in an aerobic manner under the culturing conditions of (i) pH: 5.5 to 7.5, (ii) stirring method: 5 to 40 times per minute, (iii) temperature conditions: 30 to 40°C, and (iv) culturing time: 6 to 24 hours, and extracting viable bacteria from the culture solution.

2. A method of culturing according to Claim 1, wherein the culture medium composition in said culturing step contains (i) glucose: 1 to 4%, (ii) yeast extract : 0.3 to 6%, (iii) peptone : 0.1 to 5%, and (iv) dibasic potassium phosphate, (K₂HPO₄) : 1 to 5%.

3. A method of culturing according to Claim 1 or Claim 2, wherein said bacterial cells belonging to the *Enterococcus* genus are viable bacteria selected from *Enterococcus faecalis, Enterococcus faecium, Enterococcus bovis, Enterococcus avium, Enterococcus durans, Enterococcus salivarius, Enterococcus mitis,* and *Enterococcus equines,* and mixtures of two or more thereof.

4. A method of producing killed bacterial cells belonging to the *Enterococcus* genus comprising:
(1) culturing bacterial cells of the *Enterococcus* genus in an aerobic manner under the following culturing conditions:
(i) a pH of 5.5 to 7.5;
(ii) a stirring rate of 5 to 40 revolutions per minute;
(iii) a temperature of 30 to 40°C; and
(iv) a culturing period of from 6 to 24 hours;
(2) washing resulting viable bacterial cells with physiological aqueous saline solution; and
(3) treating a bacterial liquid containing said viable bacterial cells suspended in a physiological aqueous saline solution under the following conditions:
(a) a temperature of from 80 to 125°C;
(b) a pressure of from 5 to 25 pounds per square inch [34.73 to 172.37 kPa]; and
(c) a period of from 5 to 25 minutes; thereby to kill said viable bacterial cells.

5. A method of producing a cholesterol and triglyceride reducing agent according to Claim 4, wherein the culture medium composition in said culturing step (1) contains (i) glucose: 1 to 4%, (ii) yeast extract: 0.3 to 6%, (iii) pepton: 0.1 to 5%, and (iv) dibasic potassium phosphate (K₂HPO₄) : 1 to 5%.

6. A method of producing a cholesterol and triglyceride reducing agent according to Claim 4 or Claim 5, wherein the bacterial cells belonging to the *Enterococcus* genus are viable bacteria selected from *Enterococcus faecalis, Enterococcus faecium, Enterococcus bovis, Enterococcus avium, Enterococcus durans, Enterococcus salivarius, Enterococcus mitis,* and *Enterococcus equines,* and mixtures of two more thereof.
